# EUROPÄISCHE PATENTSCHRIFT

(11) **EP 1 232 004 B1**
(45) Veröffentlichungstag und Bekanntmachung des Hinweises auf die Patenterteilung: **16.06.2004**
(21) Anmeldenummer: 00975991.1
(22) Anmeldetag: 03.11.2000
(51) Int. Cl.: B01J 8/02, C07C 57/05, B01J 19/24

(54) **VERFAHREN ZUR KATALYTISCHEN GASPHASENOXIDATION ZU (METH)ACROLEIN UND/ODER (METH)ACRYLSÄURE**
METHOD FOR CATALYTIC GAS PHASE OXIDATION TO (METH)ACROLEIN AND/OR (METH)ACRYLIC ACID
PROCEDE D'OXYDATION CATALYTIQUE EN PHASE GAZEUSE PERMETTANT D'OBTENIR DE L'ACIDE (METH)ACROLEINE ET/OU (METH)ACRYLIQUE

(30) Priorität: 03.11.1999 DE 19952964
(43) Veröffentlichungstag der Anmeldung: 21.08.2002
(73) Patentinhaber: BASF AKTIENGESELLSCHAFT, 67056 Ludwigshafen (DE)
(72) Erfinder: HECHLER, Claus, 67063 Ludwigshafen (DE); MACHHAMMER, Otto, 68163 Mannheim (DE); OLBERT, Gerhard, 69221 Dossenheim (DE); STABEL, Uwe, 43006 Tarragona (ES); ZEHNER, Peter, 67071 Ludwigshafen (DE)
(74) Vertreter: Isenbruck, Günter
(86) Internationale Anmeldenummer: PCT/EP2000/010851
(87) Internationale Veröffentlichungsnummer: WO 2001/032301

(56) Entgegenhaltungen:
- EP-A- 0 976 446
- DE-A- 2 016 614
- DE-A- 10 031 347
- DE-A- 19 753 720
- US-A- 4 544 544

## Beschreibung

Die Erfindung betrifft ein Verfahren zur kontinuierlichen Gasphasenoxidation zu (Meth)acrolein und/oder (Meth)acrylsäure. Der Begriff (Meth)acrolein steht im folgenden für "Acrolein oder Methacrolein", analog bezeichnet (Meth)acrylsäure die Substanzen Acrylsäure oder Methacrylsäure.

(Meth)acrolein und (Meth)acrylsäure sind bedeutende Grundchemikalien.

Die bekannten großtechnischen Verfahren zur Gasphasenoxidation zu (Meth)acrolein und/oder (Meth)acrylsäure (beispielsweise nach DE-A-196 24 31), werden in der Regel in Rohrbündelreaktoren durchgeführt, die eine große Anzahl, von zur Zeit bis zu 28.000, zwischen Rohrböden eingeschweißte Reaktionsrohre aufweisen. Vorzugsweise wird hierbei bei Temperaturen zwischen 200 und 450°C und gegebenenfalls erhöhtem Druck gearbeitet. Die Reaktionsrohre sind mit dem heterogenen Oxidationskatalysator gefüllt und werden vom Reaktionsgemisch durchströmt. Der Reaktorinnenraum zwischen den Reaktionsrohren ist von einem Wärmetauschmittel durchströmt, in der Regel eine Salzschmelze, häufig eine Mischung aus Kaliumnitrat, Natriumnitrit und Natriumnitrat, das die Reaktionswärme abführt.

Derartige Salzschmelzen können im Temperaturbereich von etwa 200 bis 480°C drucklos d.h. unter hydrostatischem Druck als primärer Wärmeträger zur Wärmeabfuhr verwendet werden. Das Salz wird zwischen Reaktoreintritt und Reaktoraustritt in der Regel im Mittel um ca. 2°C aufgeheizt. Durch Erzeugung von Dampf mit einem Druck von etwa 20 bis 60 bar wird das Salz in einem sekundären Kühlkreis auf die Zulauftemperatur zurückgekühlt.

Zur Erzielung eines guten Wärmeübergangskoeffizienten durch eine ausreichend hohe Strömungsgeschwindigkeit des Salzes um die Rohre wird das Salz im Reaktor im Querstrom zu den Rohren mit mehrfacher Umlenkung sowie im Gegenstrom zum Reaktionsgas geführt. Hierdurch sind im Reaktor radiale Temperaturunterschiede in der Salzschmelze bis zu 7°C vorhanden.

Die Temperaturregelung des Reaktors erfolgt über die Salzeintrittstemperatur, die zur Vermeidung von Totaloxidation so niedrig gewählt werden muß, daß am Ort der höchsten Temperatur im Reaktor, dem Hotspot, die gewünschte, reguläre Reaktion noch optimal abläuft.

Derartige Reaktoren haben somit zum einen den Nachteil, daß infolge der Radialströmung des Wärmetauschmittels keine Isothermie über den Reaktorquerschnitt erreicht werden kann, sondern stets radiale Temperaturdifferenzen in der Salzschmelze zwischen den innen gelegenen Reaktionsrohren im Vergleich zu weiter außen gelegenen Reaktionsrohren auftreten. Dies macht sich insbesondere im Hotspot-Bereich negativ bemerkbar mit der Folge, daß die Maximaltemperatur, bei der der Reaktor insgesamt betrieben werden kann, begrenzt werden muß, wofür hohe Mengenströme an Wärmetauschmittel notwendig sind.

Ein weiterer Nachteil ist, daß der Durchmesser von Rohrbündelreaktoren durch Abmessungen und Gewicht der Rohrböden eine technische Obergrenze hat. Die Kapazität der Anlage kann somit nur durch Vergrößern der Reaktorlänge erhöht werden. Mit zunehmender Höhe der Katalysatorschüttung in den Reaktorrohren nimmt jedoch der Druckverlust zu mit ungünstigen Auswirkungen auf die Selektivität.

Das bekannte Verfahren in Rohrbündelreaktoren hat den weiteren Nachteil, daß bei sehr hohen Umsätzen die Selektivität aufgrund der Temperaturinhomogenitäten stark absinkt. Zum Erreichen einer insgesamt hohen Ausbeute ist es dagegen anzustreben, alle Reaktionsrohre gleichförmig bei einem optimalen Wert von Umsatz und Selektivität zu betreiben.

Durch Ablagerungen von Nebenprodukten auf dem Katalysator kommt es dazu, daß außen liegende Rohre im Verlauf des Betriebs einen erhöhten Druckverlust aufweisen. Hierdurch ergibt sich eine uneinheitliche Durchströmung der Rohre, was niedrigere Umsatzraten in den äußeren Rohren zur Folge hat. Zusätzlich zum Umsatzverlust in diesen außen liegenden Rohren kommt es zu einer erhöhten Durchströmung der innen liegenden Rohre, so daß dort der Druckverlust steigt und die Ausbeute sinkt. Auch wenn nur Ablagerungen am Anfang eines Rohres vorhanden sind, wird die Reaktionsrate der Katalysatorschüttung auf der gesamten Rohrlänge beeinträchtigt.

Die DE-C-197 54 185 beschreibt beispielsweise einen Reaktor mit einem zylinderförmigen Reaktorbehälter, wobei im Reaktorbehälter als Thermobleche ausgebildete Wärmetauscherplatten in vertikaler Orientierung auf dem Siebboden des Reaktors nebeneinander. mit vorgegebenem Abstand voneinander angeordnet sind. Die Platten werden von einem Kühlmedium durchströmt, das im Bereich der Behälterdecke über geeignete Einrichtungen den Wärmetauscherplatten zugeführt und im Bereich des Behälterbodens über geeignete Einrichtungen aus den Wärmetauscherplatten abgeführt wird. Zwischen den Wärmetauscherplatten wird im Gegenstrom zum Kühlmedium ein gasförmiges Reaktionsmedium, mit Zuführung im Bereich des Behälterbodens und Abführung im Bereich der Behälterdecke geleitet. Die Druckschrift gibt jedoch keinen Hinweis darauf, daß ein derartiger Reaktor besonders vorteilhaft für die katalytische Gasphasenoxidation zu (Meth)acrolein und/oder (Meth)acrylsäure eingesetzt werden kann, wobei insbesondere die Selektivität der Reaktion zum jeweiligen Hauptprodukt gegenüber der Durchführung der Reaktion in einem Rohrbündelreaktor verbessert ist.

Die DE-A-197 19 375 beschreibt ein Verfahren zur Herstellung von Ethylenoxid durch katalytische Gasphasenoxidation von Ethylen mit Sauerstoff in einem Reaktor wobei der Katalysator in Reaktionszonen zwischen Wärmetauscherplatten angeordnet ist und vom gasförmigen Reaktionsgemisch durchströmt wird. Bei der katalytischen Gasphasenoxidation zu Ethylenoxid wird eine vergleichsweise geringe Wärmemenge je Volumeneinheit des Katalysators entwickelt.

Aus DE-A 20 16 614 ist ein zylindrischer Reaktor zur Durchführung von Gasphasenoxidationen bekannt, zum Beispiel für die katalytische Oxidation des Propylens zu Acrolein und Acrylsäure, wobei der Reaktorquerschnitt in Kreisringsektoren aufgeteilt ist, die alternierend von Reaktionsgemisch und Kühlflüssigkeit durchströmt werden.

Es ist Aufgabe der Erfindung, ein Verfahren zur Herstellung (Meth)acrolein und/oder (Meth)acrylsäure zur Verfügung zu stellen, das sich durch eine erhöhte Wirtschaftlichkeit, insbesondere bezüglich des Wärmetauschmittelaufwandes sowie durch eine verbesserte Selektivität auch bei sehr hohen Umsätzen sowie bei Anlagen mit großer Kapazität kennzeichnet.

Die Erfindung geht aus von einem Verfahren zur kontinuierlichen Gasphasenoxidation von C₃- oder C₄-Vorläufern zu (Meth)acrolein und/oder (Meth)acrylsäure in Gegenwart eines Katalysators in einem Reaktor mit Zuführung für das Reaktionsgemisch an einem Reaktorende und Abführung am gegenüberliegenden Reaktorende sowie mit im Reaktorinnenraum angeordneten Wärmetauscherplatten zur Abführung der Reaktionswärme, die von einem Wärmetauschmittel durchströmt sind.

Die Lösung ist dadurch gekennzeichnet, dass die Abführung der Reaktionswärme durch Siedekühlung unter Druck erfolgt.

In überraschender Weise wurde gefunden, daß die katalytische Gasphasenoxidation zu (Meth)acrolein und/oder (Meth)acrylsäure trotz der gegenüber der Ethylenoxidherstellung wesentlich höheren Wärmeentwicklung je Volumeneinheit Katalysator und der starken Hot-spot-Problematik, die bei der Ethylenoxidherstellung von geringer Bedeutung ist, in einem Reaktionsraum zwischen Wärmetauscherplatten und somit einer zweidimensional, über den Reaktorquerschnitt ausgedehnten Katalysatorschüttung, durchgeführt werden kann und die Reaktion dennoch beherrschbar bleibt. Dabei wurde eine nicht vorhersehbare Selektivitätssteigerung der Bildung von (Meth)acrolein und/oder (Meth)acrylsäure erreicht.

Als Ausgangsverbindungen können grundsätzlich alle geeigneten C₃- bzw. C₄-Edukte, insbesondere C₃- bzw. C₄-Alkanen, -Alkenen, -Alkanolen und/oder -Alkanalen und/oder Vorstufen hiervon, besonders vorteilhaft von Propen, Acrolein, tert.-Butanol, Isobuten, Isobutan, Isobutyraldehyd, Methacrolein, Isobuttersäure oder Methyl-tert.-butylether hergestellt. Weiterhin können alle Vorstufen der genannten Verbindungen verwendet werden, bei denen sich die eigentliche C₃-/C₄-Ausgangsverbindung erst intermediär während der Gasphasenoxidation bildet. Beispielhaft genannt für die Herstellung der Methacrylsäure sei Methyl-tert.-butylether oder Isobuttersäure.

Besonders vorteilhaft ist die katalytische Gasphasenreaktion von Propen und/oder Acrolein zu Acrylsäure mit molekularem Sauerstoff.

Vorzugsweise wird hierbei bei Temperaturen zwischen 200 und 450°C und ggf. erhöhtem Druck gearbeitet. Vorzugsweise werden als heterogene Katalysatoren oxidische Mehrkomponenten-Katalysatoren auf der Basis der Oxide von Molybdän, Bismut und Eisen in der 1. Stufe (Oxidation von Propen zu Acrolein) und der Oxide von Molybdän und Vandium in der 2. Stufe (Oxidation von Acrolein zu Acrylsäure) eingesetzt. Wird Propan als Ausgangsstoff verwendet, so kann dieses zu einem Propen-/Propan-Gemisch umgesetzt werden durch katalytische Oxidehydrierung, wie in US-A-5,510,558 beschrieben, durch homogene Oxidehydrierung, entsprechend in CN-A-1 105 352 oder durch katalytische Dehydrierung, wie in EP-A-0 253 409 beschrieben. Bei Einsatz eines Propen-/Propan-Gemischs wirkt Propan als Verdünnungsgas. Geeignete Propen-/Propan-Gemische sind auch Raffineriepropen (70 % Propen und 30 % Propan) oder Crackerpropen (95 % Propen und 5 % Propan). Grundsätzlich können Propen-/Propan-Gemische wie die oben genannten mit Sauerstoff oder Luft oder einem Gemisch aus Sauerstoff und Stickstoff jeder Zusammensetzung zu Acrolein und Acrylsäure oxidiert werden.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure, Formaldehyd, weitere Aldehyde und Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% Maleinsäureanhydrid und 20 bis 98 Gew.-%, vorzugsweise 50 bis 98 Gew.-% inerte Verdünnungsgase. Als inerte Verdünnungsgase sind insbesondere gesättigte C₁-C₆-Kohlenwasserstoffe, wie 0 bis 90 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Verdünnungsgas, enthalten.

Die Methacrylsäure kann analog zu Acrylsäure durch katalytische Gasphasenreaktion von C₄-Ausgangsverbindungen mit molekularem Sauerstoff hergestellt werden. Besonders vorteilhaft ist die Methacrylsäure z. B. durch katalytische Gasphasenoxidation von Isobuten, Isobutan, tert.-Butanol, Isobutyraldehyd, Methacrolein oder Methyl-tert.-butylether erhältlich. Als Katalysatoren werden ebenfalls übergangsmetallische Mischoxidkatalysatoren (z. B. Mo, V, W und/oder Fe) verwendet. Besonders geeignete Verfahren sind solche, bei denen die Herstellung ausgehend von Methacrolein erfolgt, insbesondere dann, wenn das Methacrolein durch gasphasenkatalytische Oxidation von tert.-Butanol, Isobutan oder Isobuten oder durch Umsetzung von Formaldehyd mit Propionaldehyd gemäß EP-B-0 092 097 erzeugt wird. Somit besteht auch die Möglichkeit, Methacrylsäure zweistufig herzustellen durch (1) Kondensation von Propionaldehyd mit Formaldehyd (in Gegenwart eines sekundären Amins als Katalysator) zu Methacrolein und (2) anschließende Oxidation des Methacroleins zu Methacrylsäure.

Ebenso wie bei der Herstellung der Acrylsäure wird nicht reine Methacrylsäure, sondern ein gasförmiges Gemisch erhalten, das neben der Methacrylsäure als Nebenkomponenten im wesentlichen nicht umgesetztes Methacrolein und/oder Wasserdampf, Kohlenmonoxid, Kohlendioxid, Stickstoff, Sauerstoff, Essigsäure, Propionsäure, weitere Aldehyde und Maleinsäureanhydrid enthalten kann. Das erfindungsgemäße Verfahren wird insbesondere dann eingesetzt, wenn das Reaktionsgemisch 0,02 bis 2 Gew.-% Methacrolein bezogen auf das gesamte Reaktionsgemisch und ansonsten im wesentlichen die gleichen entsprechenden Bestandteile wie bei der Herstellung der Acrylsäure enthält.

Die Form des Reaktors ist grundsätzlich keinen Einschränkungen unterworfen, es können übliche zylindrische Reaktoren, jedoch auch quaderförmige Reaktoren eingesetzt werden.

Ebenso bestehen keine Einschränkungen bezüglich der Ausrichtung der Reaktoren; die Reaktoren können grundsätzlich in jeder Position ausgerichtet sein, wobei für den Sonderfall der zylindrischen Reaktoren eine vertikale Ausrichtung in der Regel bevorzugt ist.

Für das Verfahren zur kontinuierlichen Gasphasenoxidation zu (Meth)acrolein und/oder (Meth)acrylsäure werden Reaktoren eingesetzt, die mit Wärmetauscherplatten ausgestattet sind.

Wärmetauscherplatten sind überwiegend flächenförmige Gebilde, die einen mit Zu- und Abführleitungen versehenen Innenraum mit geringer Dicke im Verhältnis zur Fläche aufweisen. Sie werden in der Regel aus Blechen, häufig aus Stahlblechen, hergestellt. Je nach Anwendungsfall, insbesondere den Eigenschaften des Reaktionsmediums sowie des Wärmetauschmittels können jedoch spezielle, insbesondere korrosionsfeste, Werkstoffe zum Einsatz kommen. Die Zu- bzw. Abführeinrichtungen für das Wärmetauschmittel sind in der Regel an einander entgegengesetzten Enden der Wärmetauschplatten angeordnet; bei der Reaktoren mit Wärmetauscherplatten für die Durchführung von Reaktionen mit hoher Wärmetönung, wobei es sich gleichermaßen um exotherme wie auch um endotherme Reaktionen handeln kann.

Bezüglich der in dem erfindungsgemäßen Verfahren einsetzbaren Wärmetauschmittel gibt es keine grundsätzlichen Einschränkungen. Es können sowohl anorganische wie auch organische flüssige Wärmetauschmittel eingesetzt werden, die bei der Reaktionstemperatur der katalytischen Gasphasenoxidation teilweise oder ganz verdampfen.

Erfindungsgemäß wird ein Wärmetauschmittel eingesetzt, das bei der Reaktionstemperatur der katalytischen Gasphasenoxidation zumindest teilweise verdampft. Besonders bevorzugt ist hierfür Wasser. Durch Ausnutzung der Siedekühlung wird hierbei eine effiziente Wärmeabführung gewährleistet, wobei für die Abführung der selben Wärmemenge gegenüber dem Einsatz eines Wärmetauschmittels, das seinen Aggregatzustand nicht ändert, eine wesentliche Einsparung der erforderlichen Menge erreicht wird.

Bei Anordnung des Katalysators in einer Schüttung um die Wärmetauscherplatten ist von Vorteil, daß sich das Reaktionsgas im Falle von lokalen Verengungen nach den Vorbeiströmen an der Verengung wieder auf den vollen Katalysatorquerschnitt zwischen zwei Wärmetauscherplatten verteilen kann und der gesamte Reaktionsquerschnitt zum Umsatz beitragen kann.

Siedekühlung kann sowohl im Gleich- wie auch im Gegenstrom erfolgen. Bei Betrieb im Gleichstrom mit Anströmung von unten besteht zusätzlich die Möglichkeit, den Stand der siedenden Flüssigkeit so zu regulieren, daß gegen Ende der Reaktionsrohre eine geringere Wärmeabfuhr erfolgt und durch das dort nun höhere Temperaturniveau die Gesamtausbeute erhöht wird.

Bei Siedekühlung stellt sich auf der Kühlmediumseite entsprechend der Temperatur ein definierter Dampfdruck ein (bei Wasser Werte im Bereich von ca. 20 bis 120 bar), so daß eine entsprechend druckfeste Auslegung der Kühlmediumseite des Apparats erforderlich ist.

Erfindungsgemäß wird das Reaktionsgemisch an einem Reaktorende dem Reaktorinnenraum zwischen den Wärmetauscherplatten zugeführt und am entgegengesetzten Reaktorende abgeführt. Das Reaktionsgemisch durchströmt somit den Reaktor durch den Zwischenraum zwischen den Wärmetauscherplatten. Dadurch findet eine ständige Quervermischung des Reaktionsgemisches statt mit der Folge einer hohen Homogenität des selben. Dadurch kann bei vorgegebenem Umsatz eine wesentlich bessere Selektivität gegenüber einer Verfahrensführung erreicht werden, die eine Quervermischung nicht gewährleistet, wie es beispielsweise bei der Durchführung der Reaktion in den Reaktionsrohren eines Rohrbündelreaktors der Fall ist.

Bezüglich der Anordnung der Wärmetauscherplatten im Reaktor gibt es grundsätzlich keine Einschränkungen; die Wärmetauscherplatten können beispielsweise spiralförmig, konzentrisch oder radial im Reaktor angeordnet sein.

Besonders bevorzugt ist eine Gleichstromführung von Wärmetauschmittel und Reaktionsgemisch durch den Reaktor. Dadurch wird eine bessere Anpassung an das Temperaturprofil der Reaktion, mit Hotspot in einem früheren Reaktionsstadium, gewährleistet.

Besonders vorteilhaft werden Wärmetauscherplatten eingesetzt, die keilförmig ausgebildet sind, d.h. deren vom Wärmetauschmittel durchströmter Innenraum bevorzugt kontinuierlich in Richtung des Reaktionsgemischstromes abnimmt. Derartige keilförmige Wärmetauscherplatten können beispielsweise hergestellt werden, indem zwei Bleche übereinander gelegt und in zunehmend größeren Abständen verschweißt werden. Die Platten werden anschließend in eine leicht geneigte Aufblasvorrichtung eingeklemmt und auf einen vorgegebenen Abstand aufgeblasen.

Mittels keilförmig ausgebildeter Wärmetauscherplatten kann die Anpassung an das Temperaturprofil der Reaktion optimiert werden.

In einer weiteren vorteilhaften Ausgestaltung können die Wärmetauscherplatten vollständig oder partiell längsverschweißt sein. Dazu werden jeweils zwei Bleche übereinander gelegt, durch Rollnahtschweißung über Längsnähte verschweißt und mittels einer geeigneten Aufblasvorrichtung aufgeblasen.

Gemäß einer weiteren Ausführungsvariante sind im Reaktorinnenraum und denselben im wesentlichen vollständig ausfüllend ebene, rechteckige, parallel zueinander ausgerichtete Bleche eingebracht wobei an jedem Blech jeweils zwei gegenüberliegende Seiten in dieselbe Richtung rechtwinklig abgekantet sind und beim jeweils darauffolgenden Blech die anderen beiden einander gegenüberliegenden Seiten in dieselbe Richtung um den selben Abstand rechteckig abgekantet sind, dergestalt, daß jeweils quaderförmige Räume entstehen, wobei die jeweils benachbarten Räume im Querstrom vom Reaktionsgemisch beziehungsweise vom Wärmetauschmittel durchströmt werden.

Gemäß einer weiteren Ausgestaltung sind die Wärmetauscherplatten in Längsrichtung des Reaktors parallel zueinander angeordnet.

Der Katalysator kann in Form von Katalysatorkörpern in den Zwischenraum zwischen den Wärmetauscherplatten eingeführt werden. Das Einbringen und der Wechsel der Katalysatorschüttung ist dabei einfacher und gleichmäßiger gegenüber dem Einfüllen in die Reaktionsrohre eines Rohrbündelreaktors. Es entstehen größere zusammenhängende Reaktionsräume und die Verstopfungsgefahr der Katalysatorschüttung ist kleiner. Dies führt zu gleichmäßigen Umsätzen und damit höherer Selektivität.

Es ist jedoch auch möglich, zusätzlich oder alternativ zur Katalysatorschüttung die Wärmetauscherplatten an ihren vom Reaktionsgemisch überströmten Außenseiten katalytisch zu beschichten. Wegen der im wesentlichen ebenen Form der Wärmetauscherplatten können diese im Vergleich zu Reaktionsrohren einfacher beschichtet werden.

Gemäß einer weiteren bevorzugten Ausführungsvariante können zwei oder mehrere Reaktionszonen mit getrennten Wärmetauschmittelkreisläufen in Richtung des Reaktionsgemischstromes angeordnet sein. Eine derartige Verfahrensvariante ist für die zweistufe Oxidation des C₃-Edukts zu Acrolein in einer ersten Verfahrensstufe mit Abführung der Reaktionswärme über einen ersten Stapel von Wärmetauscherplatten, Zwischenkühlung über einen zweiten Stapel von Wärmetauscherplatten und schließlich in der zweiten Oxidationsstufe zu Acrylsäure und Wärmeabführung über einen dritten Stapel von Wärmetauscherplatten besonders geeignet. Es ist jedoch auch möglich, unter Verzicht auf die Zwischenkühlung in einem Apparat mit lediglich zwei Stapeln von Wärmetauscherplatten die zweistufe Oxidation zu Acrylsäure durchzuführen.

Das erfindungsgemäße Verfahren hat somit den Vorteil, daß durch Einsatz von Kühlflächen mit außen liegender katalytischer Schüttung das Kühlmedium im Gleich- oder Gegenstrom zum Reaktionsgas geführt werden kann. Hierdurch wird im gesamten Reaktor in den einzelnen Reaktorquerschnitten eine Temperaturgleichverteilung erzielt, so daß die Hotspot-Temperatur über den gesamten Reaktorquerschnitt gleich ist, was zu verbesserter Ausbeute führt.

Besonders vorteilhaft ist die Verfahrensvariante mit Kühlung im Gleichstrom, weil die Temperatur des Kühlmediums bei Erreichen des Hotspots noch niedrig und die Kühlung dadurch sehr effektiv ist, wodurch der umzupumpende Mengenstrom an Kühlmittel erheblich reduziert werden kann.

Die weitere Verfahrensvariante, wonach als Kühlmedium eine siedende Flüssigkeit, insbesondere Wasser, eingesetzt wird, weist zusätzliche Vorteile auf: die Wärmeabfuhr wird erheblich verbessert und bei Verwendung von Wasser ist zusätzlich kein sekundäres Kühlmedium erforderlich.

Bevorzugt können die Wärmetauscherplatten zu zwei oder mehreren Modulen zusammengefaßt werden. Die Module sind dabei so anzuordnen, daß der Reaktorquerschnitt möglichst weitgehend gleichförmig ausgefüllt wird. Sie können in unterschiedlicher, bevorzugt jedoch in untereinander gleicher geometrischer Ausgestaltung, vorzugsweise in keilförmiger Geometrie, ausgebildet sein. Der modulartige Aufbau ist insbesondere für große Reaktoren von Vorteil, da aus fertigungstechnischen Gründen die maximale verfügbare Breite einzelner Wärmetauscherplatten begrenzt ist. Diese Begrenzung kann durch Ausbildung mehrerer Plattenmodule überwunden werden. Vorteilhaft ist weiterhin, daß die Plattenmodule einzeln austauschbar sind und das ein gezielter Katalysatorwechsel in einzelnen Modulen möglich ist.

In besonders vorteilhafter Weise ermöglicht die erfindungsgemäße Lösung eine Hochlastfahrweise im Verfahren zur kontinuierlichen Gasphasenoxidation zu (Meth)acrolein und/oder (Meth)acrylsäure. Erfindungsgemäß wird die Reaktionswärme durch ein in Wärmetauscherplatten strömendes Wärmetauschmittel abgeführt. Wärmetauscherplatten können relativ eng zueinander angeordnet werden. Mit abnehmenden Plattenabstand, im Bereich von etwa 10 bis 30 mm, vorzugsweise von 15 bis 20 mm, kann das Reaktionsmedium besser gekühlt werden, die Wärmeabführung wird verbessert, der Hotspot abgesenkt mit der Folge, daß der Durchsatz und somit die Belastung des Reaktors gegenüber einem herkömmlichen Rohrbündelreaktor erhöht werden können.

Die Erfindung wird im folgenden anhand einer Zeichnung näher erläutert.

In den Figuren werden gleiche oder entsprechende Merkmale mit gleichen Bezugsziffern versehen.
- Fig. 1: stellt eine besonders bevorzugte Ausführungsform eines zur Durchführung des Verfahrens besonders geeigneten Reaktors im Längsschnitt dar,
- Fig. 1A: einen Querschnitt durch den Reaktor aus Fig. 1,
- Fig. 1B: einen Längsschnitt durch eine Wärmetauscherplatte des Reaktors aus Fig. 1,
- Fig. 1C: eine bevorzugte Anordnung der Schweißstellen der Wärmetauscherplatte aus Fig. 1b,
- Fig. 2: einen Längsschnitt durch einen für die Durchführung des Verfahrens besonders geeigneten Reaktor mit Gleichstromführung von Reaktionsgemisch und Wärmetauschmittel,
- Fig. 3: einen Längsschnitt durch eine weitere bevorzugte Ausführungsform eines für die Durchführung des Verfahrens besonders geeigneten Reaktors mit Gegenstromführung von Reaktionsgemisch und Wärmetauschmittel,
- Fig. 4A: einen vergrößerten Ausschnitt aus dem in Fig. 4 dargestellten Reaktor zur Verdeutlichung der Bauweise der Reaktorplatten,
- Fig. 4B: einen Querschnitt durch den in Fig. 4 dargestellten Reaktor und
- Fig. 5: einen Längsschnitt durch einen Reaktor, der beispielhaft drei Reaktionszonen aufweißt,
- Fig.6A: einen Querschnitt durch einen Reaktor mit fünf Modulen von Wärmetauscherplatten und
- Fig. 6B: einen Längsschnitt durch den in Fig. 6A im Querschnitt dargestellten Reaktor.

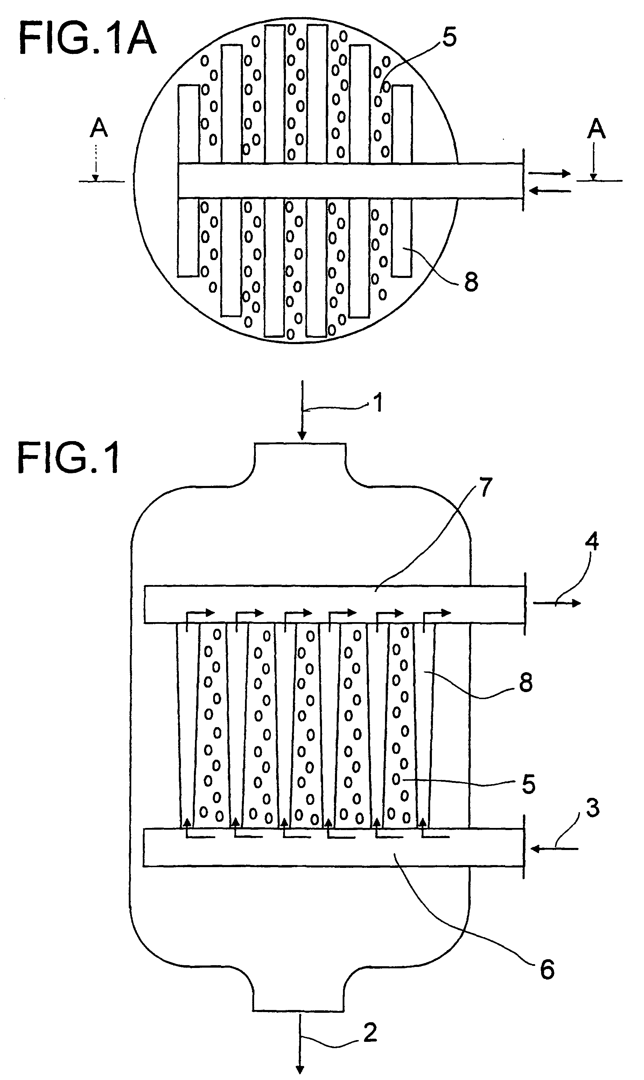

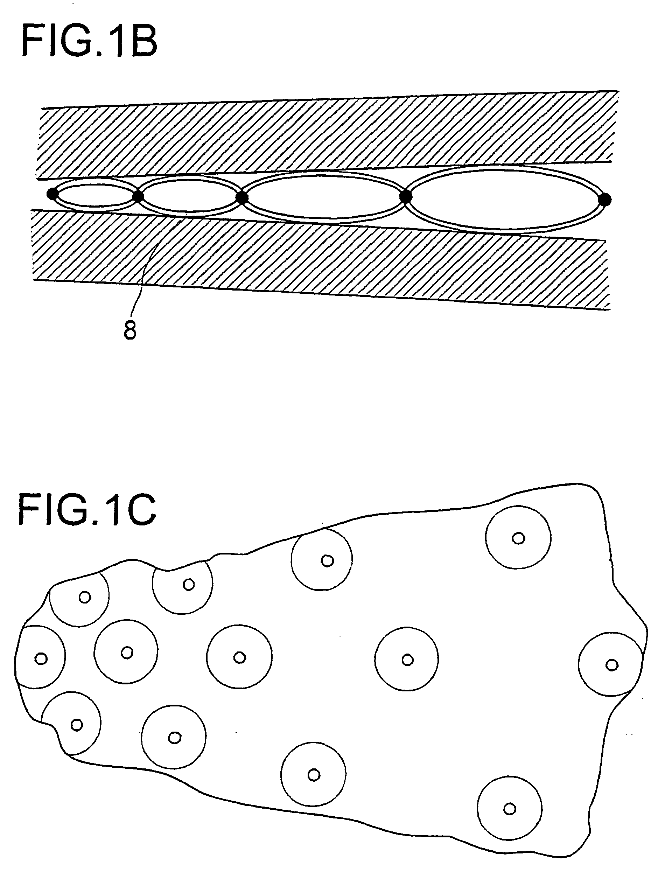

Bei der Fig. 1 im Längsschnitt dargestellten Reaktor hat die Form eines Zylinders mit Zuführung des Reaktionsgemisches 1 im oberen Bereich und Abführung des Reaktionsgemisches 2 im unteren Reaktorbereich. Das bevorzugt gasförmige Reaktionsgemisch selbst ist mit 5 bezeichnet. Im Reaktorinnenraum sind in Reaktorlängsrichtung Wärmetauscherplatten 8 angeordnet, die die besonders bevorzugte keilförmige Ausbildung aufweisen. Die Reaktorplatten werden von einem Wärmetauschmittel durchströmt, das über eine Zuführung 3 und eine Verteilerleitung 6 eingebracht und über eine Sammelleitung 7 und eine Abführleitung 4 abgeführt wird. Der Querschnitt in Fig. 1a verdeutlicht die im wesentlichen parallele Anordnung der Wärmetauscherplatten 8.

Die Fig. 1b und 1c machen die bevorzugte keilförmige Ausbildung der Wärmetauscherplatten 8 sowie deren Ausbildung durch miteinander punktverschweißte Bleche deutlich.

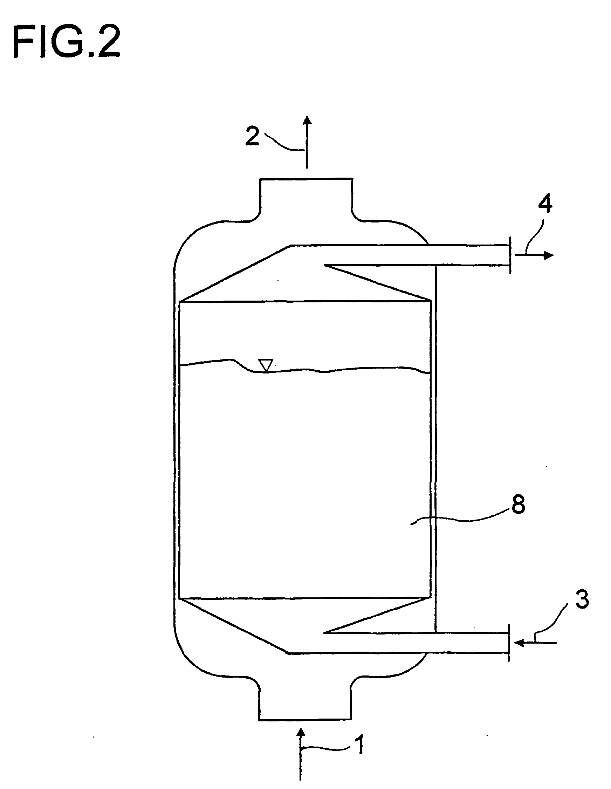

Fig. 2 zeigt beispielhaft einen Längsschnitt durch einen Reaktor mit Gleichstromführung von Reaktionsgemisch und Wärmetauschmittel. Die Fig. 2 verdeutlicht, daß in den Wärmetauscherplatten 8 der Flüssigkeitsspiegel des Wärmetauschmittels lediglich bis zu einer bestimmten Höhe steht, das Wärmetauschmittel also darüber verdampft. Die Wärmeabführung findet somit durch Siedekühlung statt.

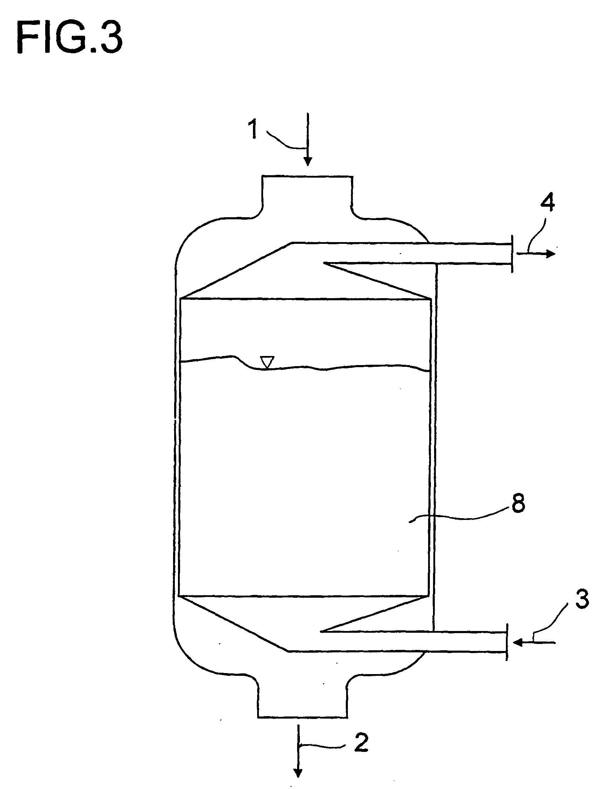

In Fig. 3 ist beispielhaft eine Gegenstromführung von Reaktionsgemisch und Wärmetauschmittel dargestellt.

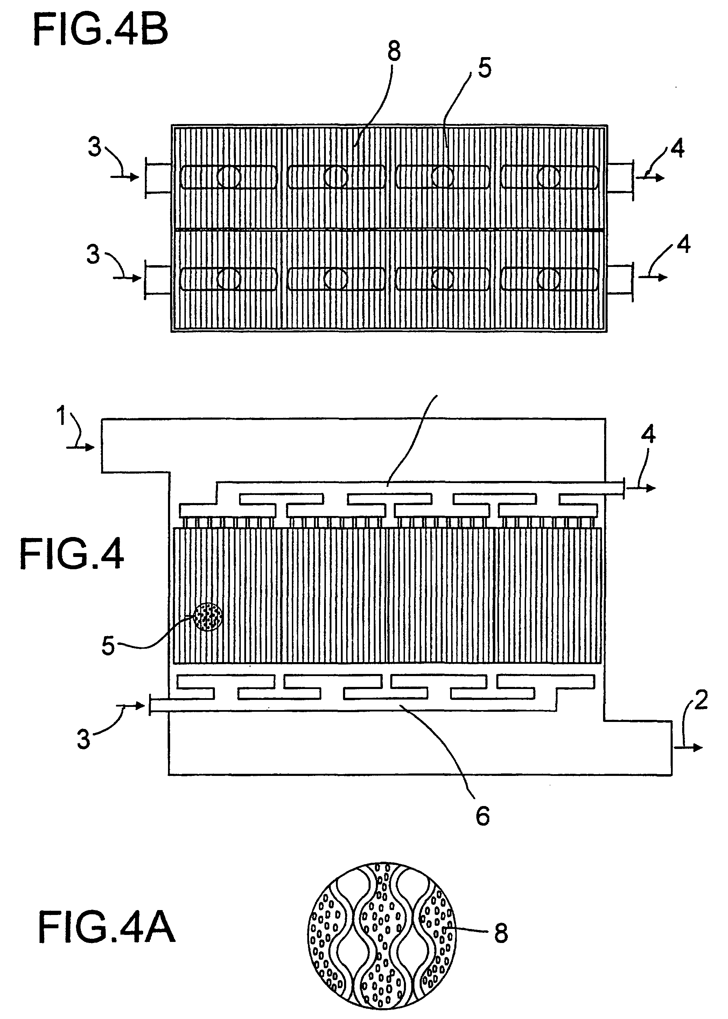

Fig. 4 zeigt einen Längsschnitt durch einen quaderförmigen Reaktor; die Ausbildung der Wärmetauscherplatten (8) ist im in Fig. 4a vergrößert dargestellten Ausschnitt verdeutlicht. Fig. 4b zeigt einen Querschnitt durch den in Fig. 4 dargestellten quaderförmigen Reaktor.

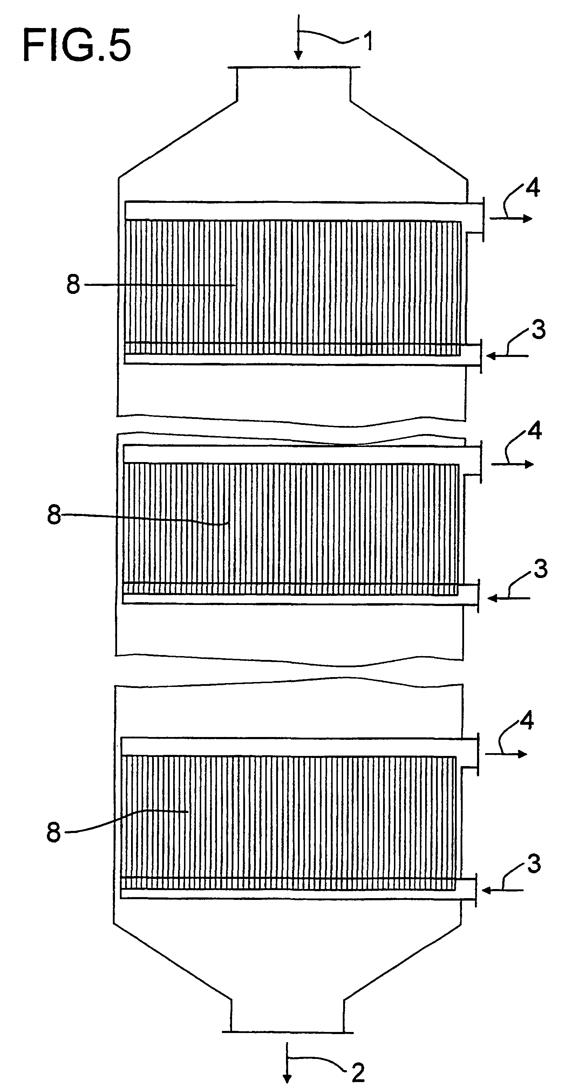

Der in Fig. 5 im Längsschnitt dargestellte Reaktor weist beispielhaft drei Reaktionszonen mit jeweils getrennten Wärmetauschmittelkreisläufen auf.

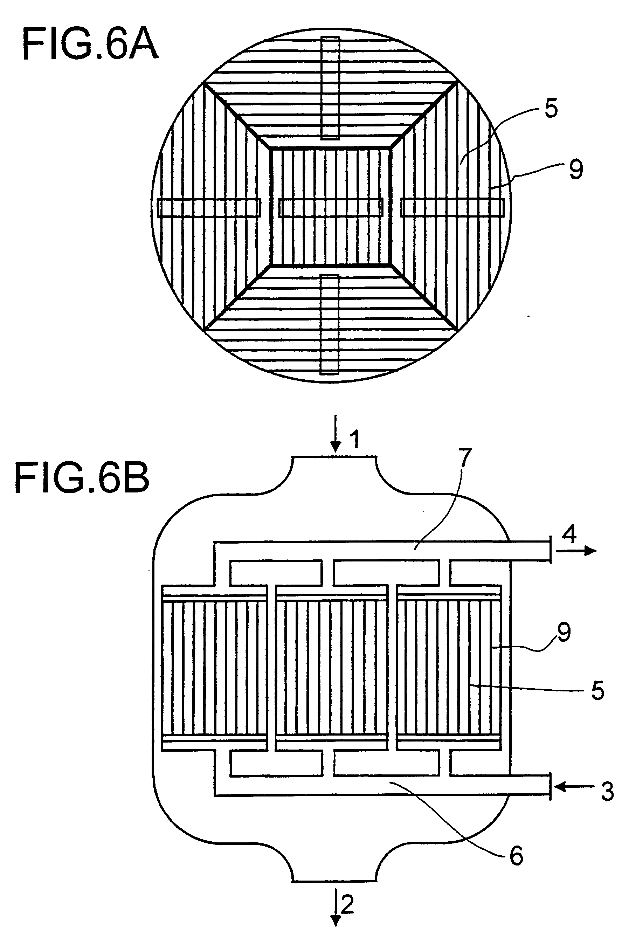

Der in Fig. 6A im Querschnitt und in Fig. 6B im Längsschnitt dargestellte Reaktor weist beispielhaft fünf zu Modulen 9 zusammengefaßte Gruppen von Wärmetauscherplatten 8 auf. Die Darstellung in Fig. 6A zeigt, daß der Querschnitt weitgehend gleichförmig mit Wärmetauscherplatten ausgefüllt ist.

## Patentansprüche

1. Verfahren zur kontinuierlichen Gasphasenoxidation von C₃- oder C₄-Vorläufern zu (Meth)acrolein und/oder (Meth)acrylsäure in Gegenwart eines Katalysators in einem Reaktor mit Zuführung (1) für das Reaktionsgemisch an einem Reaktorende und Abführung (2) am entgegengesetzten Reaktorende sowie mit im Reaktorinnenraum angeordneten Wärmetauscherplatten (8), die von einem Wärmetauschmittel durchströmt sind, zur Abführung der Reaktionswärme, **dadurch gekennzeichnet, dass** die Abführung der Reaktionswärme durch Siedekühlung unter Druck erfolgt.

2. Verfahren nach Anspruch 1, **gekennzeichnet durch** eine Gleichstromführung von Reaktionsgemisch und Wärmetauschmittel.

3. Verfahren nach Anspruch 1 oder 2, **gekennzeichnet durch** eine keilförmige Ausbildung der Wärmetauscherplatten (8), wobei der von Wärmetauschmittel durchströmte Platteninnenraum bevorzugt kontinuierlich in Richtung des Reaktionsgemischstromes abnimmt.

4. Verfahren nach einem der Ansprüche 1 bis 3, **dadurch gekennzeichnet, dass** die Wärmetauscherplatten (8) aus vollständig oder partiell längsverschweißten oder an miteinander punktverschweißten Blechen gebildet sind.

5. Verfahren nach einem der Ansprüche 1 oder 2, **dadurch gekennzeichnet, dass** im Reaktorinnenraum und denselben im wesentlichen vollständig ausfüllend ebene, rechteckige, parallel zueinander ausgerichtete Bleche eingebracht sind, wobei an jedem Blech jeweils zwei gegenüberliegende Seiten in dieselbe Richtung rechtwinklig abgekantet sind und beim jeweils darauffolgenden Blech die anderen beiden einander gegenüberliegenden Seiten in dieselbe Richtung um denselben Abstand rechteckig abgekantet sind, dergestalt, dass jeweils quaderförmige Räume entstehen, wobei die jeweils benachbarten Räume im Querstrom vom Reaktionsgemisch beziehungsweise vom Wärmetauschmittel durchströmt werden.

6. Verfahren nach einem der Ansprüche 1 bis 4, **dadurch gekennzeichnet, dass** die Wärmetauscherplatten (8) parallel zueinander angeordnet sind.

7. Verfahren nach Ansprüchen 1 bis 6, **dadurch gekennzeichnet, dass** die dem Reaktionsgemisch zugewandten Flächen der Wärmetauscherplatten vollständig oder teilweise katalytisch beschichtet sind.

8. Verfahren nach einem der Ansprüche 1 bis 7, **gekennzeichnet durch** zwei oder mehrere in Richtung des Reaktionsgemischstromes angeordnete Reaktionszonen mit getrennten Wärmetauschmittelkreisläufen.

9. Verfahren nach einem der vorhergehenden Ansprüche, **dadurch gekennzeichnet, dass** die Wärmetauscherplatten (8) zu zwei oder mehreren Modulen zusammengefasst sind.

## Claims

1. A process for the continuous gas-phase oxidation of C₃- or C₄-precursors to (meth)acrolein and/or (meth)acrylic acid in the presence of a catalyst in a reactor with a feed (1) for the reaction mixture at one reactor end and removal (2) at the opposite reactor end and with heat exchanger plates (8) which are arranged in the interior of the reactor and through which a heat exchange composition flows, for the removal of the heat of reaction, wherein the heat of reaction is removed by evaporative cooling under superatmospheric pressure.

2. A process as claimed in claim 1, comprising cocurrent flow of reaction mixture and heat exchange composition.

3. A process as claimed in claim 1 or 2, comprising a wedge-shaped formation of the heat exchanger plates (8), the plate interior through which heat exchange composition flows preferably decreasing continuously in the direction of flow of the reaction mixture.

4. A process as claimed in any of claims 1 to 3, wherein the heat exchanger plates (8) are formed from metal sheets which are completely or partially longitudinally welded or spot-welded to one another.

5. A process as claimed in either of claims 1 and 2, wherein flat, rectangular metal sheets oriented parallel to one another are introduced into the interior of the reactor, substantially completely filling said interior, in each case two opposite sides on each metal sheet being bent over at right angles in the same direction and, in the respective subsequent metal sheet, the other two sides opposite one another being bent over at right angles in the same direction by the same distance so that in each case right parallelepiped spaces form, the reaction mixture or the heat exchange composition flowing transversely through the respective adjacent spaces.

6. A process as claimed in any of claims 1 to 4, wherein the heat exchanger plates (8) are arranged parallel to one another.

7. A process as claimed in any of claims 1 to 6, wherein those surfaces of the heat exchanger plates which face the reaction mixture are completely or partially catalytically coated.

8. A process as claimed in any of claims 1 to 7, comprising two or more reaction zones arranged in the direction of flow of the reaction mixture and having separate circulations of heat exchange composition.

9. A process as claimed in any of the preceding claims, wherein the heat exchanger plates (8) are combined into two or more modules.

## Revendications

1. Procédé d'oxydation catalytique en phase gazeuse de précurseurs en C₃ ou C₄ de (méth)acroléine ou d'acide (méth)acrylique en présence d'un catalyseur, dans un réacteur avec amenée (1) du mélange réactionnel à une extrémité du réacteur et sortie (2) à l'extrémité opposée du réacteur,, ainsi qu'avec des plaques d'échange de chaleur (8) agencées dans l'espace intérieur du réacteur, et qui sont parcourues par un milieu d'échange de chaleur pour la dissipation de la chaleur de réaction, **caractérisé en ce que** la dissipation de la chaleur de réaction est assurée par refroidissement par vaporisation sous pression.

2. Procédé selon la revendication 1, **caractérisé par** un guidage en contre-courant du mélange réactionnel et du milieu d'échange de chaleur.

3. Procédé selon la revendication 1 ou 2, **caractérisé par** une configuration trapézoïdale des plaques d'échange de chaleur (8), où l'espace intérieur des plaques, parcouru par le milieu d'échange de chaleur, diminue de préférence de manière continue dans la direction de l'écoulement du mélange réactionnel.

4. Procédé selon l'une quelconque des revendications 1 à 3, **caractérisé en ce que** les plaques d'échange de chaleur (8) sont constituées de tôles totalement ou partiellement soudées longitudinalement ou soudées par points entre elles.

5. Procédé selon l'une quelconque des revendications 1 ou 2, **caractérisé en ce que**, dans l'espace intérieur du réacteur et le remplissant essentiellement en totalité, sont agencées des tôles planes, rectangulaires, parallèles entre elles, qu'à chaque tôle deux côtés opposés sont chanfreinés à angle droit dans la même direction et que, à la tôle suivante, les deux autres côtés opposés sont à chaque fois chanfreinés à angle droit dans la même direction et sur la même distance, de manière à former à chaque fois des espaces parallélépipédiques, des espaces adjacents étant respectivement parcourus en courant transversal par le mélange réactionnel ou le milieu d'échange de chaleur.

6. Procédé selon l'une quelconque des revendications 1 à 4, **caractérisé en ce que** les plaques d'échange de chaleur (8) sont agencées parallèlement entre elles.

7. Procédé selon les revendications 1 à 6, **caractérisé en ce que** les surfaces des plaques d'échange de chaleur dirigées vers le mélange réactionnel sont totalement ou partiellement munies d'un revêtement catalytique.

8. Procédé selon l'une quelconque des revendications 1 à 7, **caractérisé par** deux ou plus de deux zones de réaction agencées dans la direction de l'écoulement du mélange réactionnel, avec des circuits séparés de milieu d'échange de chaleur.

9. Procédé selon l'une quelconque des revendications qui précèdent, **caractérisé en ce que** les plaques d'échange de chaleur (8) sont composées de deux ou de plus de deux modules.
